# EUROPEAN PATENT APPLICATION

(11) **EP 0 894 853 A1**
(43) Date of publication of application: **03.02.1999**
(21) Application number: 97112704.8
(22) Date of filing: 24.07.1997
(51) Int. Cl.: C12N 15/12, C12N 15/11, C12N 15/85, C07K 14/47, A61K 38/17, A61K 48/00, A61K 31/70, G01N 33/68, C12Q 1/68

(54) **Transcriptional silencer protein NRF, nucleic acid molecules encoding it and their use**

(71) Applicant: Gesellschaft für Biotechnologische Forschung mbH (GBF), D-38124 Braunschweig (DE)
(72) Inventor: Hauser, Hansjörg, Dr., 38124 Braunschweig (DE); Nourbakhsh, Mahtab, Dr., 38124 Braunschweig (DE)
(74) Representative: Boeters, Hans Dietrich, Dr.

(57) **Abstract**

NRF is a novel inhibitory transcription factor binding to specific DNA sequences and silencing transcriptional activity of proximal DNA-binding activators, e.g. NF-_{K}B binding sites.

NRF is a modulator protein of NF-_{K}B family members controlling genes of significant biomedical importance such as those encoding inflammatory cytokines, MHC proteins, cell adhesion molecules, and viruses. Based on this it represents a molecular target in the devolopment of novel anti-inflammatory therapies for a variety of pathologic disorders such as ischemia, hemorrhagic and septic shock, allograft rejection, bacterial meningitis, acute airway inflammation and the pulmonary complications induced by cardiopulmonary bypass. Furthermore, this might apply for certain cancers and other diseases.

NRF as a target for drugs (obtained e.g. by IITS) is protected. Agonists as well as antagonists of NRF are also protected. Further applications such as the use of NRF protein and its fusion proteins or NRF DNA sequence in sense or antisense orientation in gene therapy are also protected.

## Description

The invention concerns the transcriptional silencer protein NRF which is a novel inhibitory transcription factor, and several related subject matters. The background of the invention is as follows.

### NF-_{K}B/rel protein

The family of NF-_{K}B/rel transcription factors regulates a variety of promoters through specific DNA-binding sites. NF-_{K}B/rel-binding sites act as weak constitutive enhancers. However, many promoters which contain NF-_{K}B/rel-binding sites do not show base level activity. This is explained by the existence of silencer elements. Further to the constitutive enhancing activity of the NF-_{K}B binding sites, many inducers like viruses, TNF-α or PMA induce a signalling cascade that increases the activity of the NF-_{K}B enhancers transiently. These inducers lead to a transient inactivation of IkB, the cytoplasmic inhibitor of certain NF-_{K}B members. This results in a nuclear translocation of the prototype NF-_{K}B (a heterodimer of p50 and p65) and the activation of the above mentioned target genes by binding and activation of transcription.

The nuclear factor NF-_{K}B rel family is involved in the regulation of a number of genes which contribute to physiological activities, like inflammation and cell growth. Inflammatory pathology and cancer are often associated with the disregulation of theses genes, raising the possibility that the initiation of multiple pathologic processes is due to NF-_{K}B/rel-mediated transactivation. Inhibitors of NF-_{K}B activation may therefore have broad applications as novel therapeutics in human diseases. Natural repression mechanisms might play an important part in the control of disregulated NF-_{K}B/rel activity.

### The IFN-β negative regulatory element (NRE)

An example of a repression mechanism was found in the control of mammalian Interferon-β (IFN-β) gene expression. IFN-β genes are absolutely silent but can be transcriptionally activated in nearly all differentiated type of cells by viruses or double-stranded RNA.

In this promoter several positive regulatory domains (PRDs) are assembled within less than 100 base pairs. The PRDII, which represents a binding site for NF-_{K}B/rel proteins and PRDI to which members of the IRF-family can bind are responsible for a basal expression level (1, 9, 14, 18, 38). For full activity of the IFN-β promoter further PRD sequences are required.

A minimal virus response element (VRE) was identified. It contains PRDI, PRDII and an extended negative regulatory domain (NRD). The NRD was found to repress a basal transcriptional activity in the absence of inducers (10, 11). Within this NRD, an 11 base pair element acts as a negative regulatory element (NRE) of the PRDII sequence. Although this NRE is physically overlapping with PRDII, it can act as a position-independent silencer of PRDII. (24)

### NREs in other promoters

Examination of NF-_{K}B/rel-binding sites containing promoters for the presence of NRE-related sequences and functions revealed several elements with a loose sequence relationship to the IFN-β NRE.

In the HIV-1 promoter, two sequences with homology to the IFN-β NRE were found in a region which was functionally defined as a region of negative regulation for HIV-1 transcription (3, 22). Saksela and Baltimore (1993) have described a negatively acting element (termed _{K}NE) immediately upstream of the NF-_{K}B-binding site in the Ig_{K} intronic enhancer. The core of this 27-bp _{K}NE sequence shows a high homology to the IFN-β NRE. A negative regulatory element was shown to exist in the IL-2 receptor α chain promoter (33) also exhibiting some sequence similarity to the IFN-β NRE. Homology to NRE in the HTLV-I promoter was also found. The region in which this sequence is located cooperates positively with the 21-bp enhancer upon Tax protein activation (34). The inducibility of these promoters involves the activation of NF-_{K}B/rel binding sequences.

The NRE-related sequences contained within the promoters of HIV-1 and HTL V-1 and the IL-2R-α gene constitute functionally related silencer elements which repress the constitutive enhancing activity of NF-_{K}B/rel-binding sites from these promoters. Thus, NREs represent a new class of transcrlptional repressor sequences with a silencing activity on the constitutive activity of NF-_{K}B/rel binding sites.

All NREs show similar properties with respect to binding of proteins from nuclear extracts, however, with distinguishable affinities. The distinct affinities reflect the silencing capacity of the NREs. The NRE-mediated silencing effect is relieved by enhancer-specific inducers like viruses, TAX or PMA. Despite this homology, the NF kB/rel DNA-binding sites from HIV-1-LTR and IFN-β promoter exhibit significant differences. These differences are based on the sequence specificities of the NF-_{K}B/rel-binding sites, but not on the sequences of the NREs.

### Common features of NREs:

The common features exerted by the presently known five NREs are: sequence homology, short length (11-13 bp), distance and position-independent action, specific interaction with NF-_{K}B/rel-binding sequences and indistinguishable binding patterns of nuclear factors. A considerable number of other NREs in various genes is expected to exist.

For example, sequence comparisons show NRE homology sequences within the promoters of the cell adhesion molecules ELAM-1 and CAM-1. A negatively acting element (termed kNE) with homology to the NRE core sequence was described immediately upstream of the NF-_{K}B-binding site in the Igk intronic enhancer (27).

### Target sequence specificity of NRE-function:

The NREs do not act on the basal transcription machinery. (24) Up to now, only the NRE silencing of NF-_{K}B/rel enhancers has been found. However, other activating sequences may also be silenced by the NREs. This is supported by the identification of a silencer from the gastrin promoter (39) which is highly homologous to the described NREs. The gastrin promoter does not contain NF-_{K}B/rel or related binding sites, assuming that other enhancer(s) interact with the gastrin silencer (37).

### Proteins binding to NREs:

Sequence and functional homology of the NREs from different sources suggest that the binding factors to these NREs are distinct or identical.

In EMSA the IFN-β NRE (N) sequence is retarded to give two major complexes (bands), the faster one having a higher binding affinity than the slower migrating complex (24). All functional NRE sequences are retarded in exactly the same manner, giving rise to the two complexes. The ability to form indistinguishable complexes suggests that the factors binding to these oligonucleotides are identical. This was further confirmed by cross-competition experiments. All NREs compete with each other in the same way as by themselves. However, competition data also demonstrate that affinity within the different complexes differs. The highest affinity is exhibited by IFN-β NRE, followed by the IL-2 Rec α NRE. NREs from both HIV-1 and HTLV-I show a clearly lower affinity. The affinities of NREs to nuclear proteins roughly reflect their silencing capacity to the NF-_{K}B/rel enhancer.

UV-crosslinking data suggested that the proteins would have molecular weight(s) of about 100 KDa (24). The currently published experiments do not allow a determination of the number of factors that are involved in the NRE specific silencing function(s).

### Known NF-_{K}B/-repressors:

Recently, a nuclear NF-_{K}B/rel inhibitor was described. This factor inhibits DNA-binding of p50/p65 heterodimers in Adenovirus transformed cells (21). Obviously, this repressor acts by suppressing the induced NF-_{K}B-enhancer activity and is therefore distinct to the factor(s) which repress the constitutive NF-_{K}B-enhancer activity.

A Drosophila 43 KDa HMG1 protein called DSP1 (dorsal switch protein) was described. This protein inhibits the Dorsal enhancer by binding to a proximally located sequence (17). Furthermore, DSP-1 represses NF-_{K}B/rel-site mediated enhancement in mammalian cells. The human homologue to DSP-1 which was believed to act as the IFN-β specific silencing protein, has not yet been described.

The viral transactivator Tax of HTLV-1 is able to restore the activity of the HIV-1 NF-_{K}B enhancer silenced by any NRE. Similar to this observation, Salvetti et al. (29) described the repression of an NF-_{K}B/rel binding site in the human vimentin promoter by a negative element which would be relieved by Tax expression. Tax transactivates several promoters through the induction of NF-_{K}B/rel proteins by nuclear translocation of cytoplasmatic dimers and de novo synthesis of c-rel (16). It has been shown that Tax is able to induce nuclear translocation of NF-_{K}B/rel proteins retained in the cytoplasm through interaction with p 105 or p 100 (19, 23). Induction of NF-_{K}B/rel enhancing activity by the Tax protein would result in masking the inhibitory effect exerted by the NREs. The unresponsiveness of the IFN-β and HTLV-1 NF-_{K}B enhancers to Tax expression and thus the unaltered repression by NREs may be due to the sequence differences of the investigated enhancers. Such differences in binding and transactivation of the known NF-_{K}B/rel dimers are well documented (20).

### Release from repression:

The repressive effect of the IFN-β NRE on PRDII cannot be eliminated by viral infection although this leads to an induction of NF-_{K}B binding activity (24). The inducible derepression of PRDII is dependent on the interaction with PRDI, a binding site for IRF-proteins. Similarly, induced NF-_{K}B/rel activity due to viral infection is not sufficient to activate the HTLV-1 enhancer. Depending on the NF-_{K}B enhancer element and the inducing agent, derepression requires an additional activator. The concerted action of coactivators or the induction of a particular set of NF-_{K}B/rel binding proteins are sufficient for releasing the repression.

Virus induction does not affect the negative activity of the NRE on isolated PRDII. However, a 28 base pair fragment containing PRDI, PRDII and NRE functions as a minimal VRE indicating that it is the cooperative effect of PRDI and PRDII which is responsible for overcoming the NRE function after virus infection. These properties together with electromobility shifts and DNA-crosslinking data indicate that the proteins being responsible for the silencing effect are still bound to the NRE after transcriptional induction by virus infection. It was speculated that the silencing effect of NRE-binding factor(s) might be due to a masking of the NF-_{K}B/rel activator or to 'locking' of the basal transcriptional complex for NF-_{K}B/rel activation. It was further speculated that replacement, post-transcriptional or steric alterations of the factors bound to the PADs could eliminate the negative activity of the silencer protein(s)(24).

Apart from the activation mechanism, NF-_{K}B sequences exhibit a basal constitutive activity. Most probably, this background activity is maintained by the binding of NF-_{K}B/rel proteins which are not cytoplasmatically retained by I-_{K}B, e. g. p50 dimers. This basal activity is repressed in a number of NF-_{K}B promoters including those regulating IFN-β, IL-2 receptors-α chain.

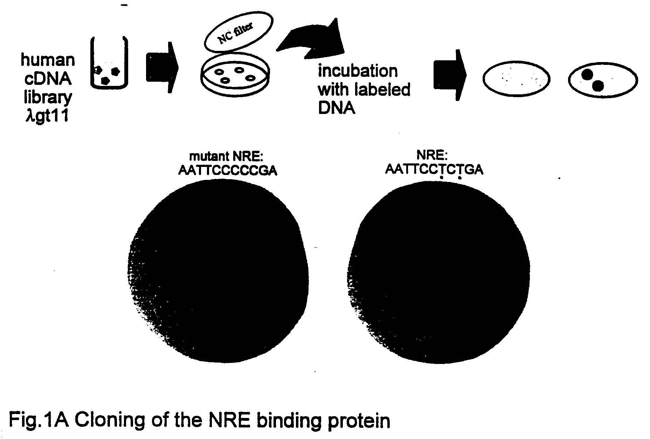

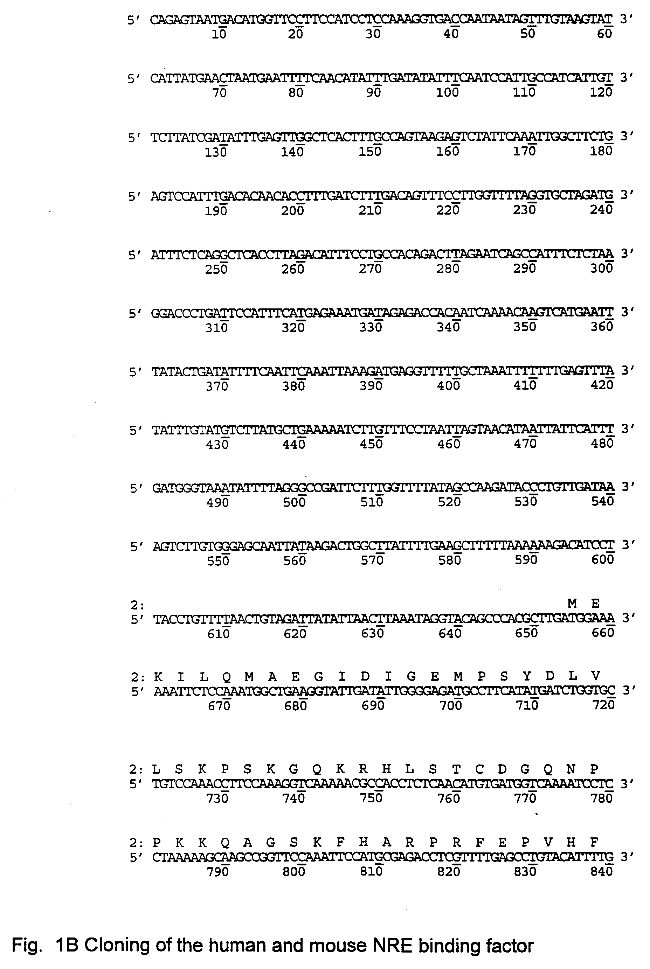

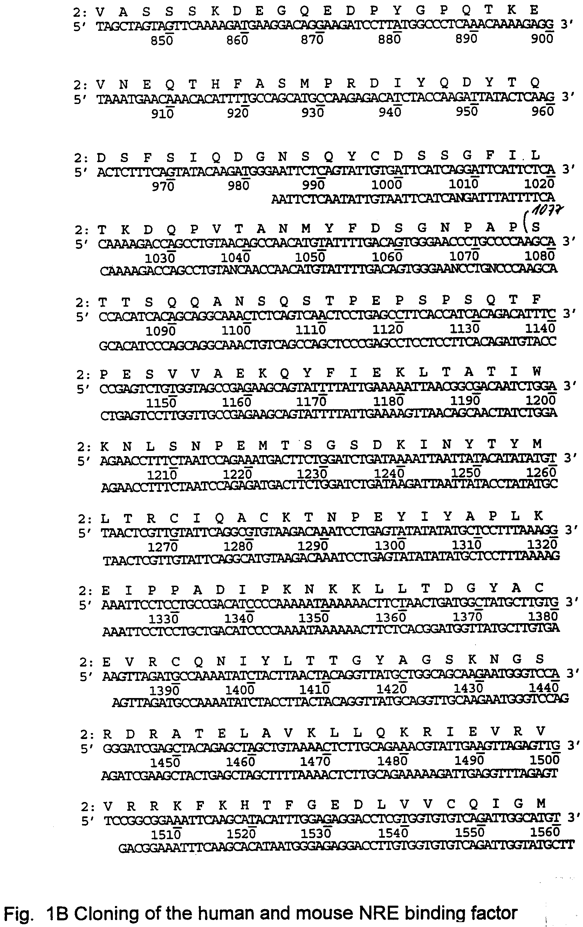

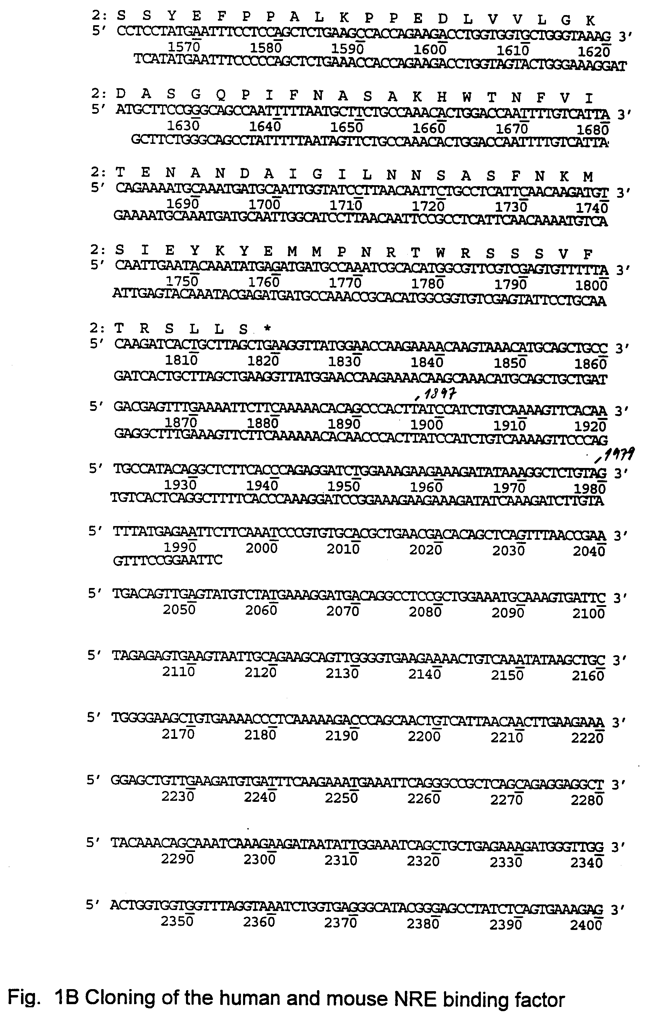

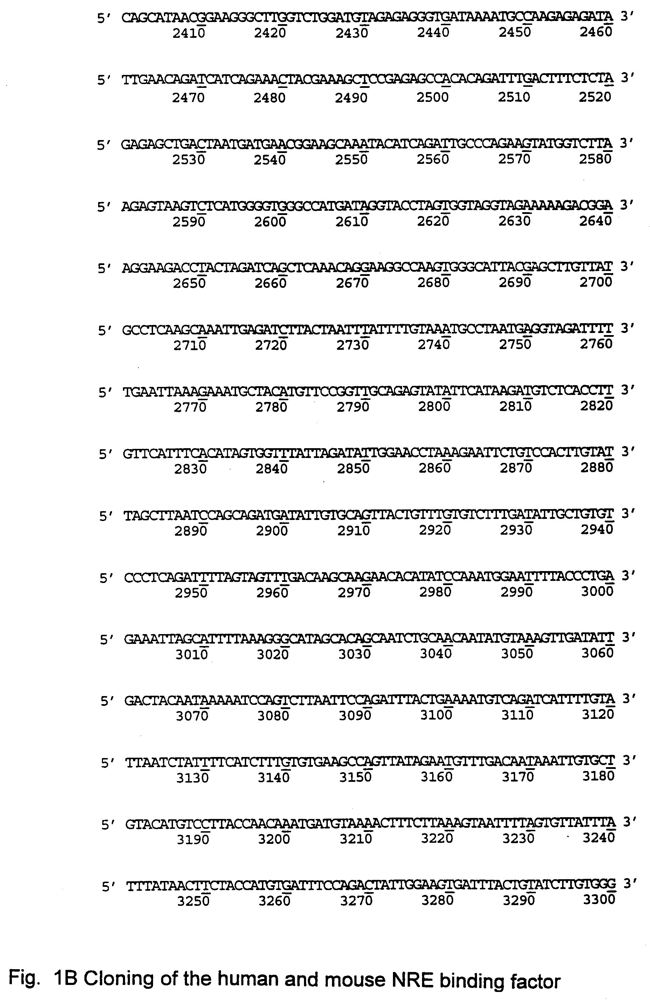

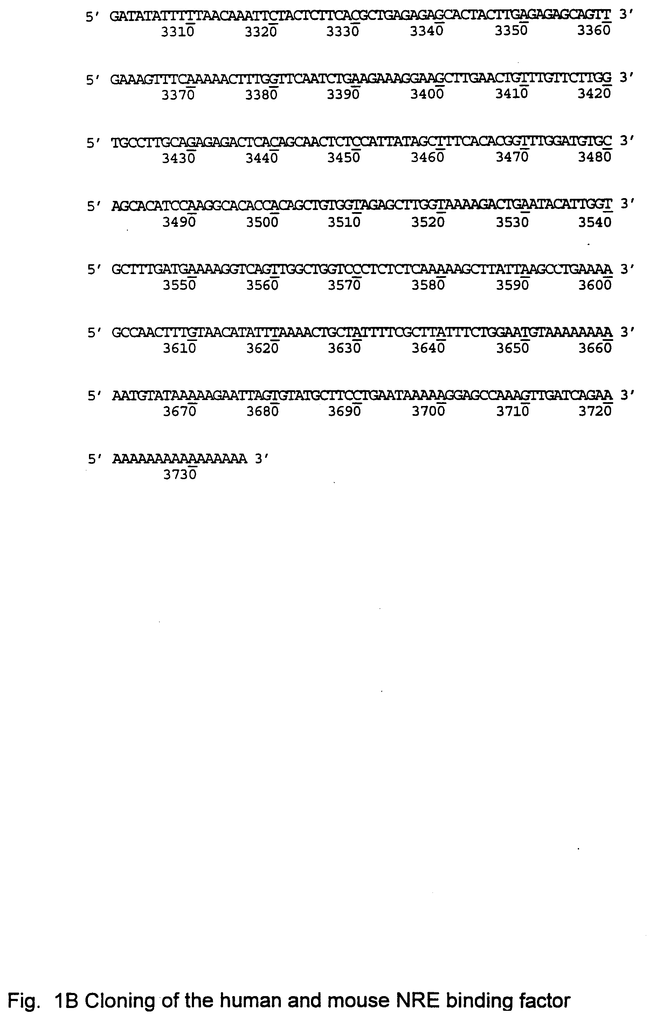

According to one embodiment the invention concerns a ssDNA
(a) having the sequence according to **Fig. 1B** or
(b) having the sequence according to **Fig. 1B** wherein
   (i) at positions 984 to 1077 and
   (ii) at positions 1897 to 1979
      the nucleotides shown in **Fig. 1B** are replaced by those shown below them in the second row or
(c1) having the same number of nucleotides as the ssDNA according to (a) or
(c2) having a reduced number of nucleotides compared with the ssDNA according to (a)
   wherein the ssDNA according to (c1) and (c2) is hybridizable with that according to (a) and/or (b).

A ssDNA according to the invention may comprise or have the nucleotide region
(i) of from position 654 to position 1817 or
(ii) of from position 1518 to 1817 (DNA binding domain = DBD) or
(iii) of from position 654 to position 1526 (silencer domain) according to **Fig. 1B** or ssDNA
(iv-i) having the same or a reduced number of nucleotides compared with the ssDNA according to (i) or
(iv-ii) having the same or a reduced number of nucleotides compared with the ssDNA according to (ii) or
(iv-iii) having the same or a reduced number of nucleotides compared with the ssDNA according to (iii)
   wherein the ssDNA according (iv-i), (iv-ii) and (iv-iii) is hybridizable with that according to (i), (ii) and/or (iii).

Another embodiment of the invention concerns a ssDNA which is characterized in that it is complementary to a ssDNA as described before.

Hybridization condition for hybridizable ssDNAs (c1), (c2) or (iv) as defined before are, for example, at a temperature of at least 25 °C and a 1 M sodium chloride concentration.

According to another embodiment the invention concerns the dsDNA consisting of a ssDNA as described above and its complementary strand.

According to another embodiment the invention concerns a RNA
(a) having a sequence corresponding to that of a DNA according to the invention as described above or
(b) having a sequence corresponding to that of a RNA according to (a) but in anti-sense or
(c) being a degradation product o a RNA according to (a) or (b) being degraded in a manner known per se.

According to another embodiment the invention concerns a vector comprising a dsDNA as described. The vector may comprise
(i) a dsDNA consisting of a ssDNA as described in paragraphs (i), (ii) and (iii) before and a complementary strand or
(ii) a ssDNA according to paragraphs (i), (ii) and (iii) as described before and a complementary strand coding the same amino acid sequence in antisense compared to (i).

A vector according to the invention may be used for the transformation of cells and organisms for transient or for permanent expression of a protein encoded by the dsDNA comprised by the vector.

Another embodiment of the invention concerns a protein encoded by a ssDNA or a dsDNA as described before, optionally fused with another functional protein or one or more functional fragments thereof. As regards these functional fragments, they may be fused with the protein according to the invention as interspiced fragments. Of course, the protein according to the invention may be an unfused protein.

Another embodiment of the invention concerns a protein (dominant negative mutant) which can be obtained by
(a) mutating the nucleotide sequence of a ssDNA according to the invention in a manner known per se,
(b) expressing the mutated ssDNA (ssDNAs) in a manner known per se
(c) subjecting the expression product(s) to a competing test for inhibition of transcription with a protein encoded by the unmodified ssDNA (starting ssDNA) and
(d) isolating a protein which acts as a dominant negative mutant of the protein encoded by the unmodified ssDNA.

A protein which represses the human IFN-β promoter was postulated earlier. However, the properties of NRF and the effects exerted upon overexpression of its sense and antisense RNA are unexpected because
- crosslinking analysis revealed not one but at least two proteins of about 100 kDa molecular mass and
- NRF has no homology to teh DSP-1 protein which was thought to be the *Drosophila* homologue to the IFN-β repressor (17).

The protein(s) according to the invention encoded by the human gene has (have) the following properties:
- It binds specifically DNA-sequences which are identical or related to the NRE-motif. The NREs are contained in a number of promoters of human genes.
- It affects transcription of a number of cellular and viral genes, e. g. it represses the background activity of NF-_{K}B/rel-binding enhancer elements. By these properties, it constitutively represses the activity of a number of cellular genes.
- Inactivation of endogenous NRE expression leads to the induction of cellular genes, i. g. the IFN-β gene.

NRF can be regarded as a modulator protein of NF-_{K}B family members controlling genes of significant biomedical importance such as those encoding inflammatory cytokines, MHC proteins, cell adhesion molecules, and viruses. Based on this it represents a molecular target in the development of novel antiinflammatory therapies for a variety of pathologic disorders such as ischemia, hemorrhagic and septic shock, allograft rejection, bacterial meningitis, acute airway inflammation and the pulmonary complications induced by cardiopulmonary bypass. Furthermore, this might apply for certain cancers and other diseases.

The following figures 1 to 8 and the following examples explain the invention in greater detail.

### Example 1

### 1. Cloning of the human and mouse NRE binding factor (Negative Regulatory Factor):

A) The method relies on the expression of human cDNA inserts from HeLa cells in bacteriophage lambda gt11. Fusion protein adsorbed onto nitrocellulose filters (NC) is probed with radioactive, double-stranded NRE-sequence as a ligand; NRE cf. Nourbakhsh et al. in EMBO J., 12 (1993) 451-459. Specific NRE-binding signals were detected on filters and corresponding bacteriophage plaques were isolated. Specific DNA-binding signals were detected on duplicate filters probed either with NRE-sequence or mutant NRE-sequence.
   A cDNA clone coding for a 44 kd protein was detected with high specificity for NRE-sequence. Bacterial cells were infected with distinct number of the recombinant bacteriophage carrying cDNA of 44 kd protein and duplicate filters were probed with labeled DNA as indicated. The filters were exposed to X-ray film overnight, generating autoradiographic images (**Fig. 1A**).
B) The human NRF cDNA was used as hybridization probe to screen for homologous sequences in a cDNA-bank from mouse embryos (day 11) at reduced stringency. Identified clones were isolated, characterized and sequenced. **Fig. 1B** shows the sequence alignment of human (middle line) and murine (underneath) NRF cDNA. The protein coding region of human NRF cDNA is given by indicated amino acid sequence above the human cDNA sequence.

### 2. Structure of the human negative regulatory factor (NRF):

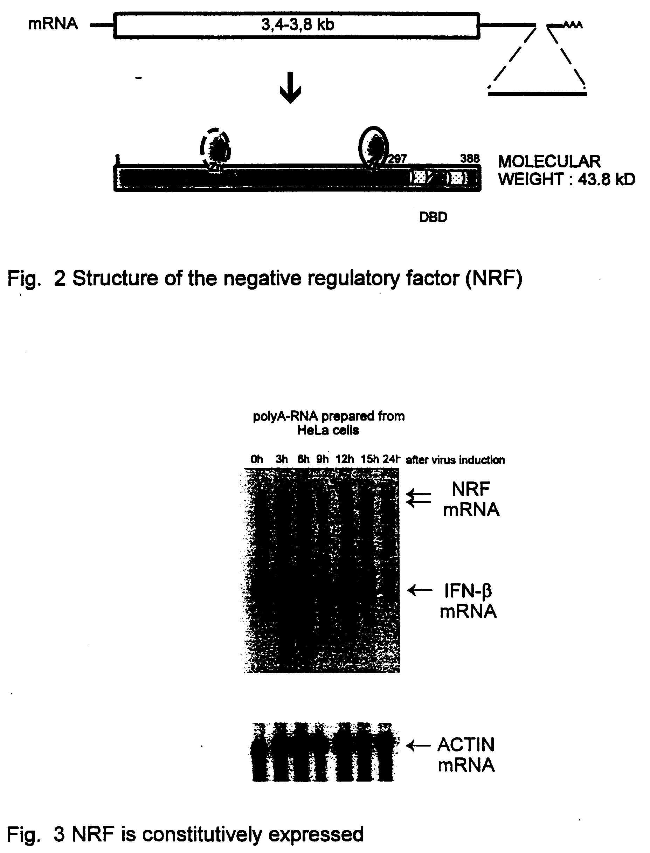

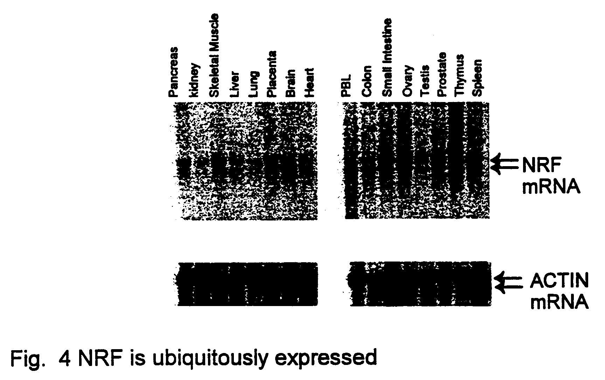

Two mRNAs with different 3'untranslated regions were identified, coding for NRF in human cells. The size of the NRF mRNAs is 2.8 and 3.8 kb (**Figs. 3 and 4**). The coding region of mRNA is indicated by an open bar in **Fig. 2**. In the same figure, the protein sequence of NRF consisting of 388 aa is demonstrated by a dark bar. The silencer domain of NRF consisting of first 291 aa contains two different zinc-fingers indicated by bubbles. The DNA binding domain of NRF (DBD) is within 100 amino acids of the C-terminal end and contains a helix-loop-helix motif (**Fig. 2**).

### 3. NRF is constitutively expressed:

The expression level of NRF was determined by Northern blot analysis using poly(A)-RNA from HeLa cells. In **Fig. 3**, two different mRNA corresponding to NRF are indicated (top). Both mRNAs are constitutively expressed and their level of expression is not altered after treatment of the cells with Newcastle disease virus. Interferon-β mRNA (middle) shows a typical induction of the cells. Actin mRNA indicates equal amounts of RNA on each lane (obtained by rehybridization).

### 4. NRF is ubiquitously expressed:

The expression level of NRF in different human tissues was determined by Northern blot analysis. Two different mRNA corresponding to NRF are indicated on the top. Actin mRNA is indicated showing an equal amount of RNA on each lane (**Fig. 4**).

### 5. NRF binds to NRE regulated promoters in vivo:

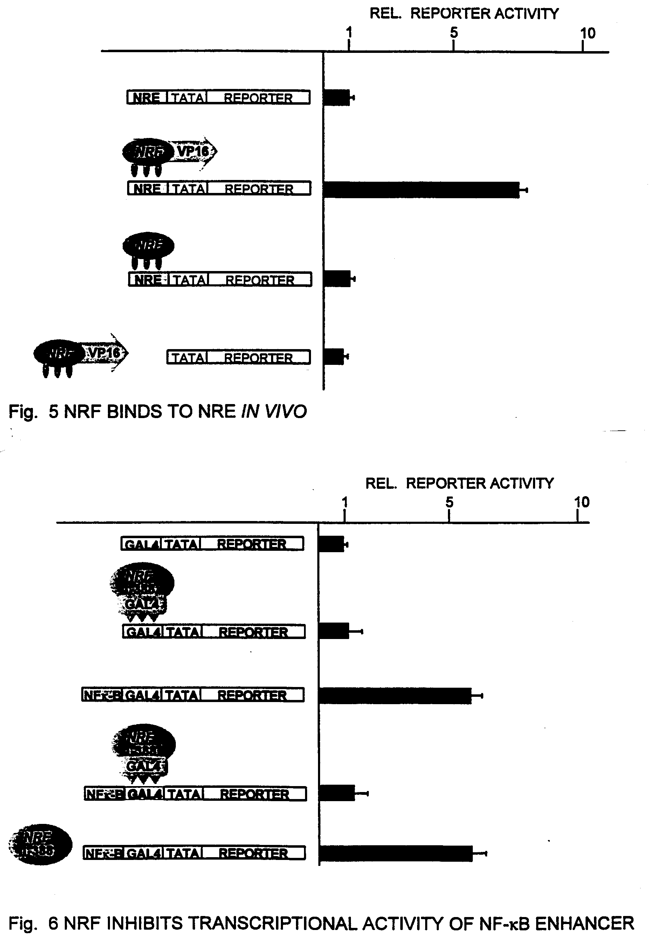

DNA-binding activity of NRF *in vivo* was tested using constructs encoding either NRF or a chimaeric protein consisting of full length NRF and the VP16 activating domain from Herpes simplex virus. Expression of these chimaeric proteins were carried out in murine cells habouring various reporter constructs as indicated by bars on the left side of **Fig. 5**. The relative reporter activities are indicated by black bars on the right side (**Fig. 5**). The data show that while w.t. NRF does not alter the expression of the NRE-promoter, the synthetic fusion protein NRF-VP16 acts as a transcriptional activator of this construct. The repressor activity of NRF is not detectable in this assay since the reporter does not contain an NF-_{K}B/rel site. The fusion protein does not affect promoters which do not contain NRE-recognition sites.

### 6. NRF inhibits transcriptional activity of NF-_{K}B promoters:

Transcriptional activity of NRF was tested using constructs encoding either NRF or a chimaeric protein consisting of NRF and the GAL4 DNA-binding domain. Expression of these chimaeric proteins was carried out in murine cells harbouring the indicated reporter constructs. These contain GAL4 and NF-_{K}B binding sites as indicated. The relative reporter activities are given as black bars on the right side (**Fig. 6**).

The fusion protein GAL4-NRF exhibits its repressor activity combined with the DNA-binding property of GAL4. The promoter in which an NF-_{K}B site enhances the basal promoter activity is inhibited by the fusion protein expression, whereas the basal promoter is not affected. W.t. NRF does not affect the reporter gene expression since its promoter does not contain an NRE.

### 7. NRF contains separable domains for silencing and DNA-binding.

The 100 C-terminal amino acids of NRF are sufficient to bind to NRE. This was demonstrated by expression in *E. coli* of an incomplete cDNA clone. The experiment was as outlined in **Fig. 1A**.

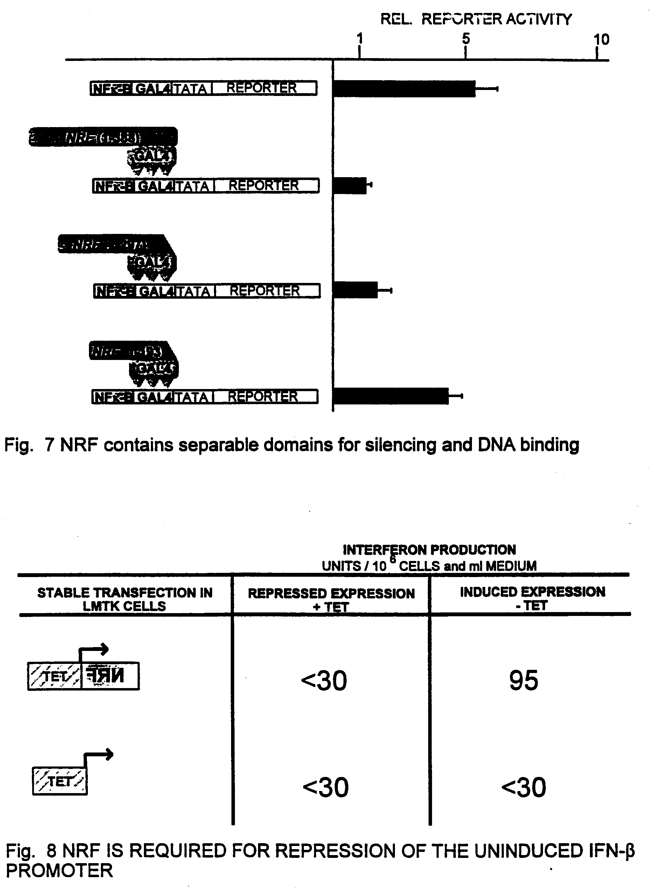

In order to define the repressor domain of NRF constructs were designed encoding chimaeric proteins containing C-terminal deletions of NRF and the GAL4 DNA-binding domain. These chimaeric proteins were expressed in murine cells harbouring a reporter construct containing GAL4 and NF-_{K}B binding sites as indicated in **Fig. 8**. The relative reporter activities are indicated by black bars.

### 8. Antisense expression of NRF affects endogenous gene expression:

A conditioned expression plasmid encoding human NRF antisense cDNA (bp 1-344, aa 1-114) was stably transferred into murine C243 cells. IFN activity in the supernatant was measured only upon induction of NRF antisense expression. This result indicates that endogenous NRF expression which represses a constitutive activity of the IFN-β promoter is eliminated by the antisense RNA.

### Example 2

An oligonucleotide having the sequence of **Fig. 1B** can also be obtained by screening material of a public gene library by means of a synthetic primer, having a subsequence according to **Fig. 1B**, in a manner non per se and by isolating the oligonucleotide wanted.

REFERENCES

1. Beg, A. A., W. C. Sha, R. T. Bronson, S. Ghosh, and D. Baltimore. 1995. Embryonic lethality and liver
degeneration in mice lacking the RelA component of NF-kappaB . Cell. 376:165-170
2. Beraud, C., S.-C. Sun, P. Ganchi, D. W. Ballard, and W. C. Greene. 1994. Human T-cell leukemia virus
type I Tax associates with and is negatively regulated by the NF-kappaB2 p100 gene product: Implications for viral
latency. Mol. Cell. Biol.. 14:1374-1382
3. Boehnlein, E., J. W. Lowenthal, M. Siekevitz, D. W. Ballard, R. Franza, and W. C. Green. 1988. The same
indiucible nuclear protein regulates mitogen activation of both the interleukin-2 receptor-alpha gene and type 1
HIV. Cell. 53:827-836
4. Colbère-Garapin, F., F. Horodniceanu, P. Khourilsky, and A. C. Garapin. 1981. A new domonant hybrid
selective marker for higher eukaryotic cells. J. Mol. Biol.. 150:1-13
5. Dignam, J. D., R. M. Lebovitz, and R. G. Roeder. 1983. Accurate transcription initiation by RNA polymerase
II in a soluble extract from isolated mammalian nuclei. Nucleic Acids Res.. 11:1475-1489
6. Drew, P. D., G. Franzoso, L. M. Carlson, W. E. Biddison, U. Siebenlist, and K. Ozato. 1995. Interferon
regulatory factor-2 physically interacts with NF-kappaB in vitro and inhibits NF-kappaB induction of major
histocompatibility class I and Beta2-microglobulin gene expression in transfected human neuroblastoma cells. J.
Neuroimmunol.. 63:157-162
7. Du, H., A. L. Roy, and R. G. Roeder. 1993. Human transcription factor USF stimulates transcription through
the initiator elements of the HIV-1 and the Ad-ML promoters. EMBO J.. 12:501-511
8. Fried, M., and D. M. Crothers. 1981. Equilibria and kinetics of lac repressor-operator interactions by
polyacrylamide gel elecrophoresis. Nucleic Acids Res.. 9:6505-6525
9. Fujita, T., Y. Kimura, M. Miyamoto, E. L. Barsoumian, and T. Taniguchi. 1989. Induction of endogenous
IFN-α and IFN-ß genes by a regulatory transcription factor, IRF-1. Nature (London). 337:270-272
10. Goodbourn, S., H. Burstein, and T. Maniatis. 1986. The human beta-interferon gene enhancer is under
negative control. Cell. 45:601-610
11. Goodbourn, S., and T. Maniatis. 1988. Overlapping positive and negative regulatory domains of the human
ß-interferon gene. Proc. Natl. Acad. Sci. USA. 85:1447-1451
12. Herschbach, B. M., and A. D. Johnson. 1993. Transcriptional repression in eukaryotes. Annu. Rev. Cell
Biol.. 9:479-509
13. Hirsch, M.-R., L. Gaugler, H. Deagostini-Bazin, and L. Bally-Cuif. 1990. Identification of Positive and
Negative Regulatory Elements Governing Cell-Type-Specific Expression of the Neural Cell Adhesion Molecule
Gene. Mol. Cell. Biol.. 10:1959-1968
14. Hiscott, J., D. Alper, L. Cohen, J. F. Leblanc, L. Sportza, A. Wong, and S. Xanthoudakis. 1989. Induction
of human interferon gene expression is associated with a nuclear factor that interacts with the NF-kappaB site of
the human immundeficiency virus enhancer. J. Virology. 63:2557-2566
15. John, S., R. B. Reeves, J.-X. Lin, R. Child, J. M. Leiden, C. B. Thompson, and W. J. Leonard. 1995.
Regulation of cell-type-specific interleukin-2 receptor Alpha-chain gene expression: Potential role of physical
interactions between Elf-1, HMG-I(Y), and NF-kappaB family proteins. Mol. Cell. Biol.. 15:1786-1796
16. Kanno, T., K. Brown, G. Franzoso, and U. Siebenlist. 1994. Kinetic analysis of human T-cell leukemia virus
type I tax-mediated activation of NF-kappaB. Mol. Cell. Biol.. 14:6443-6451
17. Lehming, N., D. Thanos, J. M. Brickman, J. Ma, T. Maniatis, and M. Ptashne. 1994. An HMG-like protein
that can switch a transcriptional activator to a repressor. Nature (London). 371:175-179
18. Lenardo, M. J., C.-M. Fan, T. Maniatis, and D. Baltimore. 1989. The involvement of NF-kappaB in ß-
interferon gene regulation reveals its role as widely inducible mediator of signal transduction. Cell. 57:287-294
19. Lindholm, P. F., M. Tamami, J. Makowski, and J. N. Brady. 1996. Human T-cell lymphotropic virus type
1 Tax1 activation of NF-kappaB: Involvement of the protein kinase C pathway. J. Virol.. 70:2525-2532
20. Liou, H.-C., and D. Baltimore. 1993. Regulation of the NF-kappaB/rel transcription factor and IkappaB
inhibitor system. Curr. Opin. Cell Biol.. 5:477-487
21. Liu, X., R. Ge, and R. P. Ricciardi. 1996. Evidence for the involvement of a nuclear NF-kappa inhibitor in
global down-regulation of the major histocompatibility complex class I enhancer in adenovirus type 12-
transformend cells. Mol. Cell. Biol.. 16:398-404
22. Lu, Y., N. Touzjian, M. Stenzel, T. Dorfman, J. G. Sodroski, and W. A. Haseltine. 1990. Identification of
cis-acting repressive sequences within the negative regulatory element of human immunodeficiency virus type 1.
J. Virol.. 64:5226-5229

23. Mielke,E., M. Nourbakhsh and H. Hauser. A new class of transcriptional repressor sequences which silence
the activity of NF-κB/rel binding sites. In preparation
24. Munoz, E., and A. Israel. 1995. Activation of NFkappaB by Tax Protein of HTLV-1. immunology. 193:128-
136
25. Nourbakhsh, M., K. Hoffmann, and H. Hauser. 1993. Interferon-ß promoters contain a DNA element that
acts as a position-independent silencer on the NF-kappaB site. EMBO J.. 12:451-459
26. Pazin, M. J., Sheridan, P. L., Cannon, K., Cao, Z. D., Keck, J. G., Kadonaga, J. T., Jones, and K. A..
1996. NF-kappaB-mediated chromatin reconfiguration and transcriptional activation of the HIV-1 enhancer in
vitro. Genes Dev.. 10:37-49
27. Reil, H., H. Kollmus, U. H. Weidle, and H. Hauser. 1993. A heptanucleotide sequence mediates ribosomal
frameshifting in mammalian cells. J. Virol.. 67:5579-5584
28. Saksela, K., and D. Baltimore. 1993. Negative regulation of immunoglobulin kappa light-chain gene
transcription by a short sequence homologous to the murine B1 repetitive element. Mol. Cell. Biol.. 13:3698-3705
29. Salvetti, A., A. Lilienbaum, Z. Li, D. Paulin, and L. Gazzolo. 1993. Identification of a negative element in
the human vimentin promoter: modulation by the human T-cell leukemia virus type I Tax protein. Mol. Cell. Biol..
13:89-97
30. Sambrook, J., E. F. Frisch, and T. Maniatis. 1989. Molecular Cloning: A Labatory Manual. Cold Spring
Harbor Laboratory, Cold Spring Harbor (New York).
31. Seiki, M., Y. Hattori, Y. Hirayama, and M. Yoshida. 1983. Human adult T-cell leukemia virus: complete
nucleotide sequence of the provirus genome integrated in leukemia cell DNA. Proc. Natl. Acad. Sci. USA.
80:3618-3622
32. Siekevitz, M., S. F. Josephs, M. Dukovich, N. Peffer, F. Wong-Staal, and W. C. Greene. 1987. Activation
of the HIV-1 LTR by T cell mitogens and the trans-activator protein of HTLV-I. Science. 238: 1575-1578
33. Sif, S., and T. Gilmore. 1994. Interaction of the v-Rell Oncoprotein with cellular Transcription Factor SP1.
J. Virol.. 68:7131-7138
34. Smith, M. R., and W. C. Greene. 1989. The same 50-kDa cellular protein binds to the negative regulatory
elements of the interleukin 2 receptor α-chain gene and the human immunodeficiency virus typ 1 long terminal
repeat. Proc. Natl. Acad. Sci. USA. 86:8526-8530
35. Tanimura, A., H. Teshima, J. Fujisawa, and M. Yoshida. 1993. A new regulatory element that augments
the Tax-dependent enhancer of human T-cell leukemia virus type 1 and cloning of cDNAs encoding its binding
proteins. J. Virol.. 67:5375-5382
36. Thanos, D., and T. Maniatis. 1992. The high mobility group protein HMG I(Y) is required for NF- kappaB-
dependent virus induction of the human IFN-Beta gene. Cell. 71:777-789
37. Thanos, D., and T. Maniatis. 1995. Virus induction of human IFNBeta gene expression requires the assembly
of an enhanceosome. Cell. 83:1091-1100
38. Tillotson, L. G., T. C. Wang, and S. J. Brand. 1994. Activation of Gastrin Transcription in pancreatic
insulinoma cells by a CACC promoter element and a 70-kDa sequence-specific DNA-binding protein. J. Biol.
Chem.. 269:2234-2240
39. Visvanathan, K. V., and S. Goodbourn. 1989. Double-stranded RNA activates binding of NF-kappaB to an
inducible element in the human beta-interferon promoter. EMBO J.. 8:1129-1138
40. Wang, T. C., and S. J. Brand. 1990. Islet Cell-specific Regulatory Domain in the Gastrin Promoter Contains
Adjacent Positive and negative DNA Elements. J. Biol. Chem.. 265:8908-8914
41. Whelan, J., P. Ghersa, R. H. van Huijsduijnen, J. Gray, G. Chandra, F. Talabot, and J. F. Delamater.
1991. An NFkB-like factor is essential but not sufficient for cytokine induction of endothelial leukocyte adhesion
molecule 1 (ELAM-1) gene transcription. Nucleic Acids Res.. 19:2645- 2653
42. Wildemann, A. G., P. Sasson-Corsi, T. Grundström, M. Zenke, and P. Chambon. 1984. Stimulation of in
vitro transcription from the SV 40 early promoter by the enhancer involves a specific trans- acting factor. EMBO
J.. 3:3129-3133

## Claims

1. ssDNA
(a) having the sequence according to **Fig. 1B** or
(b) having the sequence according to **Fig. 1B** wherein
(i) at positions 984 to 1077 and
(ii) at positions 1897 to 1979
the nucleotides shown in **Fig. 1B** are replaced by those shown below them in the second row or
(c1) having the same number of nucleotides as the ssDNA according to (a) or
(c2) having a reduced number of nucleotides compared with the ssDNA according to (a)
wherein the ssDNA according to (c1) and (c2) is hybridizable with that according to (a) and/or (b).

2. ssDNA comprising or having the nucleotide region
(i) of from position 654 to position 1817 or
(ii) of from position 1518 to 1817 (DNA binding domain = DBD) or
(iii) of from position 654 to position 1526 (silencer domain) according to **Fig. 1B** or ssDNA
(iv-i) having the same or a reduced number of nucleotides compared with the ssDNA according to (i) or
(iv-ii) having the same or a reduced number of nucleotides compared with the ssDNA according to (ii) or
(iv-iii) having the same or a reduced number of nucleotides compared with the ssDNA according to (iii)
whereas the ssDNA according to (iv-i), (iv-ii) and (iv-iii) is hybridizable with that according to (i), (ii) or (iii).

3. ssDNA, **characterized** in that it is complementary to a ssDNA according to any of the preceding claims.

4. ssDNA according to claim 1 (c1) or claim 1 (c2) or claim 2 (iv), being hybridizable at a temperature of at least 25 °C and a 1 M sodium chloride concentration.

5. dsDNA, consisting of a ssDNA according to any of the preceding claims and its complementary strand.

6. RNA
(a) having a sequence corresponding to that of a DNA according to claim 1, 2, 3, 4 or 5 or
(b) having a sequence corresponding to that of a RNA according to (a) but in anti-sense or
(c) being a degradation product of a RNA according to (a) or (b) being degraded in a manner known per se.

7. Vector, comprising a dsDNA according to claim 5.

8. Vector,
(i) comprising a dsDNA consisting of a ssDNA according to claim 2 and a complementary strand or
(ii) comprising a dsDNA consisting of a ssDNA according to claim 2 and a complementary strand coding the same amino acid sequence in antisense compared to (i).

9. Use of a vector according to claim 7 or 8 for the transformation of cells and organisms for transient or for permanent expression of a protein encoded by the dsDNA comprised by the vector.

10. Protein encoded by a ssDNA or a dsDNA according to any of claims 1 to 5, optionally fused with another functional protein or one or more functional fragments thereof.

11. Protein according to claim 10, **characterized** in that it is an unfused protein.

12. Protein (dominant negative mutant) which can be obtained by
(a) mutating the nucleotide sequence of a ssDNA according to claim 1 or 2 in a manner known per se,
(b) expressing the mutated ssDNA (ssDNAs) in a manner known per se,
(c) subjecting the expression product(s) to a competing test for inhibitation of transcription with a protein encoded by the unmodified ssDNA (starting ssDNA) and
(d) isolating a protein which acts as a dominant negative mutant of the protein encoded by the unmodified ssDNA.

13. Use of
(i) a ssDNA according to any of claims 2 to 4 or
(ii) a dsDNA according to claim 5 or
(iii) a vector according to claim 7 or 8 or
(iv) a protein according to claim 10, 11 or 12
(a) for identifying and developing agonists and antagonists of NRF-functions,
(b) for the development of improved antisense NRF and ribozymes,
(c) for the detection and diagnosis of transient or permanent regulatory disorders of NF_{K}B/rel- and/or NRF-regulated physiological patterns in animals and humans or
(d) for therapy development and treatment of diseases, especially rheumatoid, arthritis, inflammations, infectious diseases, tumors and/or genetic diseases, or
(e) for gene therapy in animals and humans.


SEQUENCE LISTING

(1) GENERAL INFORMATION:

(i) APPLICANT:
(A) NAME: Gesellschaft fuer Biotechnologische Forschung
mbH (GBF)
(B) STREET: Mascheroder Weg 1
(C) CITY: Braunschweig
(E) COUNTRY: Germany
(F) POSTAL CODE (ZIP): 38124

(ii) TITLE OF INVENTION: DNA, RNA, Vector, its use, protein
(transcriptional silencer protein NRF) and its use

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97112704.8

(2) INFORMATION FOR SEQ ID NO: 1:

(i) SEQUENCE CHARACTERISTICS:
(A) LENGTH: 3736 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(v) FRAGMENT TYPE: N-terminal

(xi) SEQUENCE DESCRIPTION: SEQ ID NO: 1:

CAGAGTAATG ACATGGTTCC TTCCATCCTC CAAAGGTGAC CAATAATAGT TTGTAAGTAT 60

CATTATGAAC TAATGAATTT TCAACATATT TGATATATTT CAATCCATTG CCATCATTGT 120

TCTTATCGAT ATTTGAGTTG GCTCACTTTG CCAGTAAGAG TCTATTCAAA TTGGCTTCTG 180

AGTCCATTTG ACACAACACC TTTGATCTTT GACAGTTTCC TTGGTTTTAG GTGCTAGATG 240

ATTTCTCAGG CTCACCTTAG ACATTTCCTG CCACAGACTT AGAATCAGCC ATTTCTCTAA 300

GGACCCTGAT TCCATTTCAT GAGAAATGAT AGAGACCACA ATCAAAACAA GTCATGAATT 360

TATACTGATA TTTTCAATTC AAATTAAAGA TGAGGTTTTT GCTAAATTTT TTTGAGTTTA 420

TATTTGTATG TCTTATGCTG AAAAATCTTG TTTCCTAATT AGTAACATAA TTATTCATTT 480

GATGGGTAAA TATTTTAGGG CCGATTCTTT GGTTTTATAG CCAAGATACC CTGTTGATAA 540

AGTCTTGTGG GAGCAATTAT AAGACTGGCT TATTTTGAAG CTTTTTAAAA AAGACATCCT 600
TACCTGTTTT AACTGTAGAT TATATTAACT TAAATAGGTA CAGCCCACGC TTGATGGAAA 660
AAATTCTCCA AATGGCTGAA GGTATTGATA TTGGGGAGAT GCCTTCATAT GATCTGGTGC 720
TGTCCAAACC TTCCAAAGGT CAAAAACGCC ACCTCTCAAC ATGTGATGGT CAAAATCCTC 780
CTAAAAAGCA AGCCGGTTCC AAATTCCATG CGAGACCTCG TTTTGAGCCT GTACATTTTG 840
TAGCTAGTAG TTCAAAAGAT GAAGGACAGG AAGATCCTTA TGGCCCTCAA ACAAAAGAGG 900
TAAATGAACA AACACATTTT GCCAGCATGC CAAGAGACAT CTACCAAGAT TATACTCAAG 960
ACTCTTTCAG TATACAAGAT GGGAATTCTC AGTATTGTGA TTCATCAGGA TTCATTCTCA 1020
CAAAAGACCA GCCTGTAACA GCCAACATGT ATTTTGACAG TGGGAACCCT GCCCCAAGCA 1080
CCACATCACA GCAGGCAAAC TCTCAGTCAA CTCCTGAGCC TTCACCATCA CAGACATTTC 1140
CCGAGTCTGT GGTAGCCGAG AAGCAGTATT TTATTGAAAA ATTAACGGCG ACAATCTGGA 1200
AGAACCTTTC TAATCCAGAA ATGACTTCTG GATCTGATAA AATTAATTAT ACATATATGT 1260
TAACTCGTTG TATTCAGGCG TGTAAGACAA ATCCTGAGTA TATATATGCT CCTTTAAAGG 1320
AAATTCCTCC TGCCGACATC CCCAAAAATA AAAAACTTCT AACTGATGGC TATGCTTGTG 1380
AAGTTAGATG CCAAAATATC TACTTAACTA CAGGTTATGC TGGCAGCAAG AATGGGTCCA 1440
GGGATCGAGC TACAGAGCTA GCTGTAAAAC TCTTGCAGAA ACGTATTGAA GTTAGAGTTG 1500
TCCGGCGGAA ATTCAAGCAT ACATTTGGAG AGGACCTCGT GGTGTGTCAG ATTGGCATGT 1560
CCTCCTATGA ATTTCCTCCA GCTCTGAAGC CACCAGAAGA CCTGGTGGTG CTGGGTAAAG 1620
ATGCTTCCGG GCAGCCAATT TTTAATGCTT CTGCCAAACA CTGGACCAAT TTTGTCATTA 1680
CAGAAAATGC AAATGATGCA ATTGGTATCC TTAACAATTC TGCCTCATTC AACAAGATGT 1740
CAATTGAATA CAAATATGAG ATGATGCCAA ATCGCACATG GCGTTCGTCG AGTGTTTTTA 1800
CAAGATCACT GCTTAGCTGA AGGTTATGGA ACCAAGAAAA CAAGTAAACA TGCAGCTGCC 1860
GACGAGTTTG AAAATTCTTC AAAAACACAG CCCACTTATC CATCTGTCAA AAGTTCACAA 1920
TGCCATACAG GCTCTTCACC CAGAGGATCT GGAAAGAAGA AAGATATAAA GGCTCTGTAG 1980
TTTATGAGAA TTCTTCAAAT CCCGTGTGCA CGCTGAACGA CACAGCTCAG TTTAACCGAA 2040
TGACAGTTGA GTATGTCTAT GAAAGGATGA CAGGCCTCCG CTGGAAATGC AAAGTGATTC 2100
TAGAGAGTGA AGTAATTGCA GAAGCAGTTG GGGTGAAGAA AACTGTCAAA TATAAGCTGC 2160
TGGGGAAGCT GTGAAAACCC TCAAAAAGAC CCAGCAACTG TCATTAACAA CTTGAAGAAA 2220
GGAGCTGTTG AAGATGTGAT TTCAAGAAAT GAAATTCAGG GCCGCTCAGC AGAGGAGGCT 2280
TACAAACAGC AAATCAAAGA AGATAATATT GGAAATCAGC TGCTGAGAAA GATGGGTTGG 2340
ACTGGTGGTG GTTTAGGTAA ATCTGGTGAG GGCATACGGG AGCCTATCTC AGTGAAAGAG 2400
CAGCATAACG GAAGGGCTTG GTCTGGATGT AGAGAGGGTG ATAAAATGCC AAGAGAGATA 2460
TTGAACAGAT CATCAGAAAC TACGAAAGCT CCGAGAGCCA CACAGATTTG ACTTTCTCTA 2520

GAGAGCTGAC TAATGATGAA CGGAAGCAAA TACATCAGAT TGCCCAGAAG TATGGTCTTA 2580

AGAGTAAGTC TCATGGGGTG GGCCATGATA GGTACCTAGT GGTAGGTAGA AAAAGACGGA 2640

AGGAAGACCT ACTAGATCAG CTCAAACAGG AAGGCCAAGT GGGCATTACG AGCTTGTTAT 2700

GCCTCAAGCA AATTGAGATC TTACTAATTT ATTTTGTAAA TGCCTAATGA GGTAGATTTT 2760

TGAATTAAAG AAATGCTACA TGTTCCGGTT GCAGAGTATA TTCATAAGAT GTCTCACCTT 2820

GTTCATTTCA CATAGTGGTT TATTAGATAT TGGAACCTAA AGAATTCTGT CCACTTGTAT 2880

TAGCTTAATC CAGCAGATGA TATTGTGCAG TTACTGTTTG TGTCTTTGAT ATTGCTGTGT 2940

CCCTCAGATT TTAGTAGTTT GACAAGCAAG AACACATATC CAAATGGAAT TTTACCCTGA 3000

GAAATTAGCA TTTTAAAGGG CATAGCACAG CAATCTGCAA CAATATGTAA AGTTGATATT 3060

GACTACAATA AAAATCCAGT CTTAATTCCA GATTTACTGA AAATGTCAGA TCATTTTGTA 3120

TTAATCTATT TTCATCTTTG TGTGAAGCCA GTTATAGAAT GTTTGACAAT AAATTGTGCT 3180

GTACATGTCC TTACCAACAA ATGATGTAAA ACTTTCTTAA AGTAATTTTA GTGTTATTTA 3240

TTTATAACTT CTACCATGTG ATTTCCAGAC TATTGGAAGT GATTTACTGT ATCTTGTGGG 3300

GATATATTTT TAACAAATTC TACTCTTCAC GCTGAGAGAG CACTACTTGA GAGAGCAGTT 3360

GAAAGTTTCA AAAACTTTGG TTCAATCTGA AGAAAGGAAG CTTGAACTGT TTGTTCTTGG 3420

TGCCTTGCAG AGAGACTCAC AGCAACTCTC CATTATAGCT TTCACACGGT TTGGATGTGC 3480

AGCACATCCA AGGCACACCA CAGCTGTGGT AGAGCTTGGT AAAAGACTGA ATACATTGGT 3540

GCTTTGATGA AAAGGTCAGT TGGCTGGTCC CTCTCTCAAA AAGCTTATTA AGCCTGAAAA 3600

GCCAACTTTG TAACATATTT AAAACTGCTA TTTTCGCTTA TTTCTGGAAT GTAAAAAAAA 3660

AATGTATAAA AAGAATTAGT GTATGCTTCC TGAATAAAAA GGAGCCAAAG TTGATCAGAA 3720

AAAAAAAAAA AAAAAA 3736

(2) INFORMATION FOR SEQ ID NO: 2:

(i) SEQUENCE CHARACTERISTICS:
(A) LENGTH: 3736 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 2:

CAGAGTAATG ACATGGTTCC TTCCATCCTC CAAAGGTGAC CAATAATAGT TTGTAAGTAT 60

CATTATGAAC TAATGAATTT TCAACATATT TGATATATTT CAATCCATTG CCATCATTGT 120

TCTTATCGAT ATTTGAGTTG GCTCACTTTG CCAGTAAGAG TCTATTCAAA TTGGCTTCTG 180
AGTCCATTTG ACACAACACC TTTGATCTTT GACAGTTTCC TTGGTTTTAG GTGCTAGATG 240
ATTTCTCAGG CTCACCTTAG ACATTTCCTG CCACAGACTT AGAATCAGCC ATTTCTCTAA 300
GGACCCTGAT TCCATTTCAT GAGAAATGAT AGAGACCACA ATCAAAACAA GTCATGAATT 360
TATACTGATA TTTTCAATTC AAATTAAAGA TGAGGTTTTT GCTAAATTTT TTTGAGTTTA 420
TATTTGTATG TCTTATGCTG AAAAATCTTG TTTCCTAATT AGTAACATAA TTATTCATTT 480
GATGGGTAAA TATTTTAGGG CCGATTCTTT GGTTTTATAG CCAAGATACC CTGTTGATAA 540
AGTCTTGTGG GAGCAATTAT AAGACTGGCT TATTTTGAAG CTTTTTAAAA AAGACATCCT 600
TACCTGTTTT AACTGTAGAT TATATTAACT TAAATAGGTA CAGCCCACGC TTGATGGAAA 660
AAATTCTCCA AATGGCTGAA GGTATTGATA TTGGGGAGAT GCCTTCATAT GATCTGGTGC 720
TGTCCAAACC TTCCAAAGGT CAAAAACGCC ACCTCTCAAC ATGTGATGGT CAAAATCCTC 780
CTAAAAAGCA AGCCGGTTCC AAATTCCATG CGAGACCTCG TTTTGAGCCT GTACATTTTG 840
TAGCTAGTAG TTCAAAAGAT GAAGGACAGG AAGATCCTTA TGGCCCTCAA ACAAAAGAGG 900
TAAATGAACA AACACATTTT GCCAGCATGC CAAGAGACAT CTACCAAGAT TATACTCAAG 960
ACTCTTTCAG TATACAAGAT GGGAATTCTC AATATTGTAA TTCATCANGA TTTATTTTCA 1020
CAAAAGACCA GCCTGTANCA ACCAACATGT ATTTTGACAG TGGGAANCCT GNCCCAAGCA 1080
CCACATCACA GCAGGCAAAC TCTCAGTCAA CTCCTGAGCC TTCACCATCA CAGACATTTC 1140
CCGAGTCTGT GGTAGCCGAG AAGCAGTATT TTATTGAAAA ATTAACGGCG ACAATCTGGA 1200
AGAACCTTTC TAATCCAGAA ATGACTTCTG GATCTGATAA AATTAATTAT ACATATATGT 1260
TAACTCGTTG TATTCAGGCG TGTAAGACAA ATCCTGAGTA TATATATGCT CCTTTAAAGG 1320
AAATTCCTCC TGCCGACATC CCCAAAAATA AAAAACTTCT AACTGATGGC TATGCTTGTG 1380
AAGTTAGATG CCAAAATATC TACTTAACTA CAGGTTATGC TGGCAGCAAG AATGGGTCCA 1440
GGGATCGAGC TACAGAGCTA GCTGTAAAAC TCTTGCAGAA ACGTATTGAA GTTAGAGTTG 1500
TCCGGCGGAA ATTCAAGCAT ACATTTGGAG AGGACCTCGT GGTGTGTCAG ATTGGCATGT 1560
CCTCCTATGA ATTTCCTCCA GCTCTGAAGC CACCAGAAGA CCTGGTGGTG CTGGGTAAAG 1620
ATGCTTCCGG GCAGCCAATT TTTAATGCTT CTGCCAAACA CTGGACCAAT TTTGTCATTA 1680
CAGAAAATGC AAATGATGCA ATTGGTATCC TTAACAATTC TGCCTCATTC AACAAGATGT 1740
CAATTGAATA CAAATATGAG ATGATGCCAA ATCGCACATG GCGTTCGTCG AGTGTTTTTA 1800
CAAGATCACT GCTTAGCTGA AGGTTATGGA ACCAAGAAAA CAAGTAAACA TGCAGCTGCC 1860
GACGAGTTTG AAAATTCTTC AAAAACACAG CCCACTTATC CATCTGTCAA AAGTTCCCAG 1920
TGTCACTCAG GCTTTTCACC CAAAGGATCC GGAAAGAAGA AAGATATCAA AGATCTTGTG 1980
TTTATGAGAA TTCTTCAAAT CCCGTGTGCA CGCTGAACGA CACAGCTCAG TTTAACCGAA 2040
TGACAGTTGA GTATGTCTAT GAAAGGATGA CAGGCCTCCG CTGGAAATGC AAAGTGATTC 2100

TAGAGAGTGA AGTAATTGCA GAAGCAGTTG GGGTGAAGAA AACTGTCAAA TATAAGCTGC 2160

TGGGGAAGCT GTGAAAACCC TCAAAAAGAC CCAGCAACTG TCATTAACAA CTTGAAGAAA 2220

GGAGCTGTTG AAGATGTGAT TTCAAGAAAT GAAATTCAGG GCCGCTCAGC AGAGGAGGCT 2280

TACAAACAGC AAATCAAAGA AGATAATATT GGAAATCAGC TGCTGAGAAA GATGGGTTGG 2340

ACTGGTGGTG GTTTAGGTAA ATCTGGTGAG GGCATACGGG AGCCTATCTC AGTGAAAGAG 2400

CAGCATAACG GAAGGGCTTG GTCTGGATGT AGAGAGGGTG ATAAAATGCC AAGAGAGATA 2460

TTGAACAGAT CATCAGAAAC TACGAAAGCT CCGAGAGCCA CACAGATTTG ACTTTCTCTA 2520

GAGAGCTGAC TAATGATGAA CGGAAGCAAA TACATCAGAT TGCCCAGAAG TATGGTCTTA 2580

AGAGTAAGTC TCATGGGGTG GGCCATGATA GGTACCTAGT GGTAGGTAGA AAAAGACGGA 2640

AGGAAGACCT ACTAGATCAG CTCAAACAGG AAGGCCAAGT GGGCATTACG AGCTTGTTAT 2700

GCCTCAAGCA AATTGAGATC TTACTAATTT ATTTTGTAAA TGCCTAATGA GGTAGATTTT 2760

TGAATTAAAG AAATGCTACA TGTTCCGGTT GCAGAGTATA TTCATAAGAT GTCTCACCTT 2820

GTTCATTTCA CATAGTGGTT TATTAGATAT TGGAACCTAA AGAATTCTGT CCACTTGTAT 2880

TAGCTTAATC CAGCAGATGA TATTGTGCAG TTACTGTTTG TGTCTTTGAT ATTGCTGTGT 2940

CCCTCAGATT TTAGTAGTTT GACAAGCAAG AACACATATC CAAATGGAAT TTTACCCTGA 3000

GAAATTAGCA TTTTAAAGGG CATAGCACAG CAATCTGCAA CAATATGTAA AGTTGATATT 3060

GACTACAATA AAAATCCAGT CTTAATTCCA GATTTACTGA AAATGTCAGA TCATTTTGTA 3120

TTAATCTATT TTCATCTTTG TGTGAAGCCA GTTATAGAAT GTTTGACAAT AAATTGTGCT 3180

GTACATGTCC TTACCAACAA ATGATGTAAA ACTTTCTTAA AGTAATTTTA GTGTTATTTA 3240

TTTATAACTT CTACCATGTG ATTTCCAGAC TATTGGAAGT GATTTACTGT ATCTTGTGGG 3300

GATATATTTT TAACAAATTC TACTCTTCAC GCTGAGAGAG CACTACTTGA GAGAGCAGTT 3360

GAAAGTTTCA AAAACTTTGG TTCAATCTGA AGAAAGGAAG CTTGAACTGT TTGTTCTTGG 3420

TGCCTTGCAG AGAGACTCAC AGCAACTCTC CATTATAGCT TTCACACGGT TTGGATGTGC 3480

AGCACATCCA AGGCACACCA CAGCTGTGGT AGAGCTTGGT AAAAGACTGA ATACATTGGT 3540

GCTTTGATGA AAAGGTCAGT TGGCTGGTCC CTCTCTCAAA AAGCTTATTA AGCCTGAAAA 3600

GCCAACTTTG TAACATATTT AAAACTGCTA TTTTCGCTTA TTTCTGGAAT GTAAAAAAAA 3660

AATGTATAAA AAGAATTAGT GTATGCTTCC TGAATAAAAA GGAGCCAAAG TTGATCAGAA 3720

AAAAAAAAAA AAAAAA 3736

(2) INFORMATION FOR SEQ ID NO: 3:

(i) SEQUENCE CHARACTERISTICS:
(A) LENGTH: 388 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO: 3:

Met Glu Lys Ile Leu Gln Met Ala Glu Gly Ile Asp Ile Gly Glu Met
1               5                   10                  15

Pro Ser Tyr Asp Leu Val Leu Ser Lys Pro Ser Lys Gly Gln Lys Arg
            20                  25                  30

His Leu Ser Thr Cys Asp Gly Gln Asn Pro Pro Lys Lys Gln Ala Gly
        35                  40                  45

Ser Lys Phe His Ala Arg Pro Arg Phe Glu Pro Val His Phe Val Ala
    50                  55                  60

Ser Ser Ser Lys Asp Glu Gly Gln Glu Asp Pro Tyr Gly Pro Gln Thr
65                  70                  75                  80

Lys Glu Val Asn Glu Gln Thr His Phe Ala Ser Met Pro Arg Asp Ile
                85                  90                  95

Tyr Gln Asp Tyr Thr Gln Asp Ser Phe Ser Ile Gln Asp Gly Asn Ser
            100                 105                 110

Gln Tyr Cys Asp Ser Ser Gly Phe Ile Leu Thr Lys Asp Gln Pro Val
        115                 120                 125

Thr Ala Asn Met Tyr Phe Asp Ser Gly Asn Pro Ala Pro Ser Thr Thr
    130                 135                 140

Ser Gln Gln Ala Asn Ser Gln Ser Thr Pro Glu Pro Ser Pro Ser Gln
145                 150                 155                 160

Thr Phe Pro Glu Ser Val Val Ala Glu Lys Gln Tyr Phe Ile Glu Lys
                165                 170                 175

Leu Thr Ala Thr Ile Trp Lys Asn Leu Ser Asn Pro Glu Met Thr Ser
            180                 185                 190

Gly Ser Asp Lys Ile Asn Tyr Thr Tyr Met Leu Thr Arg Cys Ile Gln
        195                 200                 205

Ala Cys Lys Thr Asn Pro Glu Tyr Ile Tyr Ala Pro Leu Lys Glu Ile
    210                 215                 220

Pro Pro Ala Asp Ile Pro Lys Asn Lys Lys Leu Leu Thr Asp Gly Tyr
225                 230                 235                 240

Ala Cys Glu Val Arg Cys Gln Asn Ile Tyr Leu Thr Thr Gly Tyr Ala
                245                 250                 255

Gly Ser Lys Asn Gly Ser Arg Asp Arg Ala Thr Glu Leu Ala Val Lys
            260                 265                 270

Leu Leu Gln Lys Arg Ile Glu Val Arg Val Val Arg Arg Lys Phe Lys
        275                 280                 285

His Thr Phe Gly Glu Asp Leu Val Val Cys Gln Ile Gly Met Ser Ser
    290                 295                 300

Tyr Glu Phe Pro Pro Ala Leu Lys Pro Pro Glu Asp Leu Val Val Leu
305                 310                 315                 320

Gly Lys Asp Ala Ser Gly Gln Pro Ile Phe Asn Ala Ser Ala Lys His
                325                 330                 335

Trp Thr Asn Phe Val Ile Thr Glu Asn Ala Asn Asp Ala Ile Gly Ile
            340                 345                 350

Leu Asn Asn Ser Ala Ser Phe Asn Lys Met Ser Ile Glu Tyr Lys Tyr
        355                 360                 365

Glu Met Met Pro Asn Arg Thr Trp Arg Ser Ser Ser Val Phe Thr Arg
    370                 375                 380

Ser Leu Leu Ser
385